# EUROPEAN PATENT SPECIFICATION

(11) **EP 2 203 175 B1**
(45) Date of publication and mention of the grant of the patent: **13.02.2013**
(21) Application number: 08835284.4
(22) Date of filing: 02.10.2008
(51) Int. Cl.: A61K 31/716, A61K 31/722, A61P 17/02

(54) **PREPARATION FOR WOUND HEALING AND PREVENTION OF BANDAGE ADHESION TO THE WOUND, CONTAINING CHITOSAN-GLUCAN**
CHITOSAN-GLUCAN ENTHALTENDES PRÄPARAT ZUR WUNDHEILUNG UND ZUR VERHINDERUNG DES ANHAFTENS VON VERBÄNDEN AN DER WUNDE
PRÉPARATION À BASE DE CHITOSAN-GLUCAN PERMETTANT DE SOIGNER DES BLESSURES ET D'ENPÊCHER UN PANSEMENT D'ADHÉRER À LA PLAIE

(30) Priority: 03.10.2007 CZ 20070683
(43) Date of publication of application: 07.07.2010
(73) Proprietor: Contipro Biotech s.r.o., 56102 Dolni Dobrouc (CZ)
(72) Inventor: VALENTOVÁ, Zuzana, 570 01 Litomysl (CZ); BILEROVÁ, Helena, 56301 Lanskroun (CZ); SULÁKOVÁ, Romana, 562 01 Ústí Nad Orlicí (CZ); VELEBNÝ , Vladimír, 564 01 Zamberk (CZ)
(74) Representative: Kania, Frantisek
(86) International application number: PCT/CZ2008/000117
(87) International publication number: WO 2009/043319

(56) References cited:
- WO-A-96/25437
- GB-A- 2 026 516
- JANA FRANKOVA ET AL: "The effect of hyaluronan combined with KI3 complex (Hyiodine wound dressing) on keratinocytes and immune cells" JOURNAL OF MATERIALS SCIENCE: MATERIALS IN MEDICINE, KLUWER ACADEMIC PUBLISHERS, BO, vol. 17, no. 10, 1 October 2006 (2006-10-01), pages 891-898, XP019399750 ISSN: 1573-4838
- MUZZARELLI R A A; TANFANI F; SCARPINI G: "CHELATING FILM FORMING AND COAGULATING ABILITY OF THE CHITOSAN GLUCAN COMPLEX FROM ASPERGILLUS-NIGER INDUSTRIAL WASTES" BIOTECHNOLOGY AND BIOENGINEERING, vol. 22, no. 4, 1980, pages 885-896, XP002515885

## Description

### Field of the Art

The invention relates to a preparation containing a pharmacologically suitable chitosan-glucan complex or a salt thereof in combination with one or more polysaccharides or suitable salts thereof, and optionally with an antiseptic agent, that is intended for wound healing and that, besides accelerating wound healing, is at the same time able to prevent a bandage adhesion to the wound.

### State of the Art

The current state of acute and chronic complicated wound healing is based on the use of various materials and techniques. An ideal proceeding is cleaning of the wound and its final surgical treatment (suture, skin autotransplantation and the like). However, said method of wound healing is possible for a limited number of skin defects only and relates mainly to surgical wounds and acute non-infected wounds.

The majority of more extensive wounds are infected and in case of chronic wounds, a bacterial contamination and a certain amount of necrosis are to be practically always anticipated. It is just these more extensive subacute and chronic wounds that are very complicated to treat. Nowadays, systems maintaining a permanently moist environment in extensive or chronic wound healing are preferred. Said systems derive from the theory formulated in the 60^{th}, when Mr. Winter proved that a moist wound heals as much as twice faster than a dry wound. In case of an open wound that is exposed to the air, dehydration and scab formation take place which acts as a mechanical barrier to the migrating epidermal cells. Then the healing processes must move to deeper tissues which slows them down significantly. [1]

The easiest way how to produce a moist environment are repeated re-bandages by moistened gauzes. The gauze is moistened by the physiological solution or a solution containing an antiseptic agent. Said method is demanding as it requires frequent re-bandages which are performed every 4 to 6 hours. Moreover, the method does not lead to a perfect wound disinfection and does not prevent the maceration of the skin surrounding the wound. Further, if the bandages are not moistened regularly, they dry and stick to the wound and their removal at the re-bandaging is very painful. This may be prevented by several other methods. [2]

In case the gauze is saturated with vaseline, it is the so-called greasy gauze. The vaseline may be combined with iodine and then the bandage has antimicrobial properties. A disadvantage of a greasy gauze use is that the vaseline closes the wound and necrotic parts and infection accumulates under the bandage. Problems arise in re-bandaging when removing the vaseline from the deeper wound. Therefore, said method is used for superficial wounds only. [3]

Smaller deep wounds are often covered with a plastic bandage that maintains a moist environment in the wound. Cellulose derivatives or alginate may be added to this bandage. Such bandage fulfils mainly the covering function and has no disinfecting effects. The bandage itself does not contribute to the necrotic tissue removal and does not actively support the healing. That means that it requires a cleaning of the wound in a separate step prior to the application of the substances accelerating the healing and, without a further support it is not able to maintain the wound sterile and to prevent a later development of an infection. [2]

The necrotic tissue may be removed from the wound either mechanically or by means of preparations containing enzymes, e.g. collagenases or papain, in an ointment or cream base. The tissue secretion is removed especially by means of preparations containing activated carbon. [4]

The Czech utility model No. 12015 discloses a preparation for chronic wound healing based on a suitable hyaluronic acid salt combined with iodine and potassium iodide. Said combination provides for an environment that disinfects the wound, prevents a further infection and, at the same time, ensures a good hydration. The suitable hyaluronic acid salt supports the wound healing. [5]

Also some other preparations make use of the hyaluronic acid for enhancing the healing effects, mostly in the form of its sodium salt and in combination with various growth factors. In this case, one disadvantage of the system lies in the fact that it requires cleaning of the wound in a preceding separate step. Another disadvantage consists in the absence of antiseptic agents which implies that without a further support, the preparation is not able to maintain the wound sterile and to prevent a later development of an infection. [2]

For the treatment of chronic wounds, a combination of collagen and a chemically modified cellulose is used. If there are no other substances added to this system, it is not able to ensure wound disinfection, secretion removal and the like just by itself. [2]

Special instrument therapies used for healing chronic wounds include the oxygenotherapy in hyperbaric chambers which is suitable especially for diabetic patients, and the vacuum therapy in which a porous elastic sponge is applied to the wound, the wound being exchanged in regular intervals, and the wound is covered with an impermeable foil. Then the air is sucked from the wound and the resulting vacuum provides for the wound cleaning and secretion removal. Both therapies are very instrument-demanding, suitable for highly specialised facilities only, and, moreover, they are limited just to certain diagnoses. [6, 7]

### References:

1. Winter G.D.: Formation of scab and rate of epithelialization of superficial wounds in the skin of the young domestic pig; Nature 1962,193,293
2. Ovington L.G.: Advances in wound dressings; Clinics Dermatol 2007, 25, 33
3. Queen D., Evans J.H., Gaylor J.D.S. et al.: Bum wound dressings - a review; Burns 1987, 13, 218
4. Falabella A.F.: Debridement and wound bed preparation; Dermatol Therapy 2006, 19, 317
5. Utility model No.. 12015, Industrial Property Office of the Czech Republic
6. http://www.worldwidewounds.com/2001/may/Thomas/Vacuum-Assisted-Closure.html; 5.9.07
7. http://en.wikipedia.org/wiki/Hyperbaric_medicine; 5.9.07

### Summary of the Invention

The object of the invention is to form a preparation which by applying to the wound supports indirectly the healing processes in the wound. Due to its specific composition, it drains the redundant secretion, together with the tissue mediators and enzymes supporting the healing, from the wound to the surface. At the same time, by doing this, it provides for the necessary hydration of the wound and its surroundings, without any maceration of the surrounding skin or drying and sticking to the wound. An addition of antiseptic agents prevents a further infection.

The subject-matter of the preparation for wound healing and prevention of the bandage adhesion to the wound according to the invention lies in that it contains a pharmacologically suitable chitosan-glucan complex or a salt thereof in combination with one or more other polysaccharides or their suitable salts, and optionally an antiseptic agent.

The pharmacologically acceptable chitosan-glucan complex has a dynamic viscosity of a 2.5% solution within the range between 0.1 and 100 Pa.s at the temperature of 25°C and revolutions of 0.0314 rad/s, the weight ratio of chitosan to 1,3-D-glucan is within the range from 0.01:99.99 to 99.99-0.01; the glucosamine content is within the range from 0 to 1 (n/n) and is in the free form or bound in the form of a pharmaceutically suitable salt. Suitable salts are hydrochloride, lactate, acetate, propionate, succinate, glycolate or another water-soluble salt of an acid, or a mixture thereof.

Said other polysaccharide may be e.g. hyaluronic acid, alginic acid (alginate), carboxymethyl cellulose, chitosan, oxidised cellulose, fucan, schizophyllan, carboxymethyl-, sulfoethyl- or another water-soluble 1,3-D-glucan. The other polysaccharide is in the free form or bound in the form of a pharmacologically suitable salt. It may be a single salt or a mixture thereof. The respective polysaccharides that are present as the so-called other polysaccharides have the following parameters:
The hyaluronic acid has the molecular weight within the range of 1 000 to 2 500 000 g/mol and is in the free form or in the form of a pharmacologically suitable salt which is a sodium, potassium, lithium, calcium, magnesium, zinc, cobalt, manganese salt or another salt or a mixture thereof. The alginic acid (alginate) has the molecular weight within the range of 1 000 to 1 000 000 g/mol, the ratio of D-mannuronic acid to L-guluronic acid is within the range of 0.1 to 5 (n/n) and is in the free form or in the form of a pharmacologically suitable salt which is a sodium, potassium, lithium, calcium, magnesium, zinc, cobalt, manganese salt or another salt or a mixture thereof. Carboxymethyl cellulose has the molecular weight within the range of 1 000 to 1 500 000 g/mol, the substitution degree is within the range of 0.1 to 3 (n/n) (the total amount of carboxymethyl groups to the total amount of monosaccharide units) and is in the free form or in the form of a pharmacologically suitable salt which is a sodium, potassium, lithium, calcium, magnesium, zinc, cobalt, manganese salt or another salt or a mixture thereof. Schizophyllan (β-1,3-D-glucan) has the molecular weight within the range of 1 000 to 2 000 000 g/mol. Chitosan has the molecular weight within the range of 1 000 to 1 000 000 g/mol, the deacetylation degree is within the range of 0.1 to 1 (n/n) and is in the free form or in the form of a pharmacologically suitable salt which is hydrochloride, lactate, acetate, propionate, succinate, glycolate or another water-soluble salt of an acid, or a mixture thereof. The oxidised cellulose (polyglucuronic acid) has the molecular weight within the range of 1 000 to 1 000 000 g/mol, the oxidation degree is within the range of 0.05 to 1 (n/n) and is in the free form or in the form of a pharmacologically suitable salt which is a sodium, potassium, lithium, calcium, magnesium, zinc, cobalt, manganese salt or another salt or a mixture thereof. Fucan has the molecular weight within the range of 1 000 to 500 000 g/mol, the ratio of the fucose units to the total number of monosaccharide units is at least 0.25 (n/n) and the weight fraction of the sulfo groups is at least 0.1 % by weight. Carboxymethyl-β-1,3/1,6-D-glucan has the molecular weight within the range of 1 000 to 1 500 000 g/mol, the substitution degree is within the range of 0.05 to 3 (n/n) (the total amount of carboxymethyl groups to the total amount of monosaccharide units) and is in the free form or in the form of a pharmacologically suitable salt which is a sodium, potassium, lithium, calcium, magnesium, zinc, cobalt, manganese salt or another salt or a mixture thereof. Sulfoethyl- β-1,3-D-glucan has the molecular weight within the range of 1 000 to 1 500 000 g/mol, the substitution degree is within the range of 0.05 to 3 (n/n) (the total amount of sulfoethyl groups to the total amount of monosaccharide units) and is in the free form or in the form of a pharmacologically suitable salt which is a sodium, potassium, lithium, calcium, magnesium, zinc, cobalt, manganese salt or another salt or a mixture thereof.

The antiseptic may be selected from the group of glycols (propylenglycol, 1,3-butylenglycol, hexylenglycol,...), quarternary ammonium salts (benzalconium chloride BAC, cetyltrimethylammonium bromide CTMB, cetylpyridinium chloride CPC, benzethonium chloride BZT...), biguanidine derivatives (chlorhexidine chloride/acetate/gluconate..., polyhexamethylenebiguanide PHMB, polyaminopropylbiguanide...), octenidines (octenidine chloride...), iodine complexes (iodine/potassium iodide...), bismuttribromophenate, silver, silver sulfadiazine or others.

The preparation according to the invention comprises a pharmacologically suitable chitosan-glucan complex within the concentration range of 0.01 to 100 % by weight of the dry matter, said other polysaccharide or a mixture of other polysaccharides within the concentration range of 0 to 99.99 % by weight of the dry matter and an antiseptic agent within the concentration range of 0 to 50 % by weight of the dry matter.

A more preferred embodiment of the invention is a preparation comprising a pharmacologically suitable chitosan-glucan complex within the concentration range of 40 to 90 % by weight of the dry matter, said other polysaccharide or polysaccharides within the concentration range of 10 to 60 % by weight of the dry matter and an antiseptic agent within the concentration range of 1 to 25 % by weight of the dry matter.

The preparation for wound healing and prevention of bandage adhesion to the wound is preferably in the form of a foil, lyophilizate, a foil deposited on a suitable textile or a lyophilizate deposited on a suitable textile or a combination of said forms.

The preparation according to the invention in the form of a foil or a foil deposited on a textile is prepared by dissolving the above mentioned ingredients in a sterile water and a subsequent pouring of the solution into a suitable mould or a suitable mould coated with a textile and drying. The preparation according to the invention in the form of a lyophylizate or a lyophilizate deposited on a textile is prepared by dissolving the above mentioned ingredients in a sterile water and a subsequent pouring of the solution into a suitable mould or a suitable mould coated with a textile, freezing and lyophilization.

The preparation for wound healing and prevention of bandage adhesion to the wound is either applied directly to the wound or is applied in a required amount to that side of the bandage which is then applied to the wound, or is inserted into a multi-layer bandage construction wherein it is placed directly behind the bandage contact layer made of a non-adhesive textile or a textile made of the oxidised cellulose and the bandage is applied to the wound by placing its contact layer on the wound.

Chitosan-glucan and other polysaccharides are molecules hydrophilic enough to ensure, upon its application, the secretion of the tissue liquid from the wound surroundings to the wound so as to provide for a permanently moistened environment suitable for wound healing (wound hydration). Regarding the fact that the water which moistens the wound comes to the bandage from the wound, no maceration of the wound surroundings occurs. The antiseptic agents disinfect the wound, thus ensuring a clean environment within the wound. All of these factors positively influence the granulation tissue formation, the subsequent epithelization, and therefore, the healing of the wound. The advantage thereof is the possibility of monitoring the bandage.

The influence of the preparation according to the invention comprising chitosan-glucan on the healing of an experimental wound is shown in Table 1. The abbreviations and parameters of the comprised substances used in Table 1 are as follows: CH-G...chitosan-glucan (the viscosity of the 2.5% solution at 25°C and revolutions 0.0314 rad/s is 6.8 Pa.s, the glucosamine content is 0.11 (n/n) and it is in the form of a salt of hydrochloric acid (hydrochloride)); HA...hyaluronic acid (its molecular weight is 1 700 000 g/mol and it is in the form of a sodium salt).

**Table 1**

| **Percentage (%) of the healed wound area** | | | | |
|---|---|---|---|---|
| Composition of the active ingredients in the preparation according to the invention | Healing time | | | |
| | 0 hours | 72 hours | 144 hours | 216 hours |
| control | 0.0 | 8.8 | 28.2 | 53.5 |
| 100 % CH-G | 0.0 | 34.8 | 57.6 | 85.8 |
| 50 % HA + 50 % CH-G | 0.0 | 31.1 | 56.6 | 87.3 |
| 25 % HA + 75 % CH-G | 0.0 | 40.3 | 62.6 | 91.8 |

### Preferred Embodiments of the Invention

The molecular weight of chitosan-glucan used herein is defined by means of the dynamic viscosity. The molecular weight of the other polymer substances used in this invention is the weight average molecular weight

### Example 1

1 g of chitosan-glucan (the dynamic viscosity is 2.5 Pa.s at the concentration of the solution of 2.5 %, at 25°C and revolutions of 0.0314 rad/s, the glucosamine content is 0.15 (n/n)) is dissolved in 100 ml of sterile inject water, then octenidine chloride is added in the form of a solution so that its final concentration in the solution with chitosan-glucan is 0.2 %, and this final solution is sterilised. The viscous solution is poured into a mould. The poured mixture is dried in a tray hot-air drier at 60°C for 24 hours. The foil thus prepared is inserted into a multi-layer bandage construction in such a way that it is placed directly behind the bandage contact layer which is made of a non-adhesive PAD textile having a mesh structure. Then the bandage is packed and sterilised. The bandage is intended to be applied directly to the wound.

### Example 2

0.75 g of chitosan-glucan (the dynamic viscosity is 6.8 Pa.s at the concentration of the solution of 2.5 %, at 25°C and revolutions of 0.0314 rad/s, the glucosamine content is 0.11 (n/n)) and 0.75 g of sodium hyaluronate having the molecular weight of 1 700 000 g/mol are dissolved in 50 ml of sterile inject water. 0.1 g of iodine is dissolved in a solution of 0.15 g of potassium iodide in 50 ml of sterile inject water. The solutions are prepared separately and are sterilised separately as well. After the sterilisation, they are mixed at sterile conditions. A thin layer of the resulting gel is applied directly to the wound on which a bandage is then applied.

### Example 3

1 g of chitosan-glucan (the dynamic viscosity is 8.6 Pa.s at the concentration of the solution of 2.5 %, at 25°C and revolutions of 0.0314 rad/s, the glucosamine content is 0.13 (n/n)), 0.5 g of schizophyllan having the molecular weight of 1 200 000 g/mol and 0.1 g of bismuttribromophenate are dissolved in 100 ml of sterile inject water and sterilised. The highly viscous gel is poured into a mould. The poured mixture is frozen at -18°C for 12 hours. The frozen mixture is placed into a lyophiliser and is lyophilised. The lyophilisate thus prepared is then placed into a multi-layer bandage construction in such a way that it is placed directly behind the bandage contact layer which is made of a non-adhesive PP textile having a mesh structure. Then the bandage is packed and sterilised. The bandage is intended to be applied directly to the wound.

### Example 4

0.75 g of chitosan-glucan (the dynamic viscosity is 12.0 Pa.s at the concentration of the solution of 2.5 %, at 25°C and revolutions of 0.0314 rad/s, the glucosamine content is 0.20 (n/n)), 0.5 g of sodium hyaluronate having the molecular weight of 1 700 000 g/mol, 0.25 g of carboxymethylglucan having the molecular weight of 200 000 g/mol and the substitution degree of 0.79 (n/n) and 0.1 g of PHMB are dissolved in 100 ml of sterile inject water and sterilised. The highly viscous gel is poured into a mould coated with a non-woven PES textile. The poured mixture is frozen at -18°C for 12 hours. The frozen mixture is placed into a lyophiliser and is lyophilised. The lyophilisate thus prepared, immobilised on the non-woven PES textile, is intended to be applied directly to the wound, wherein it is applied to the wound with the side without the textile.

### Example 5

1 g of chitosan-glucan (the dynamic viscosity is 20.0 Pa.s at the concentration of the solution of 2.5 %, at 25 °C and revolutions of 0.0314 rad/s, the glucosamine content is 0.05 (n/n)), 0.5 g of chitosan having afraction of glucosamine of 0.7 (n/n), 0.5 g of schizophyllan having the molecular weight of 1 550 000 g/mol and 0.05 g of chlorhexidin gluconate are dissolved in 100 ml of sterile inject water and sterilised. The highly viscous gel is poured into a mould. The poured mixture is dried in a tray hot-air drier at 60 °C for 24 hours. The foil thus prepared is then placed into a multi-layer bandage construction in such a way that it is placed directly behind the bandage contact layer which is a textile made of oxidised cellulose. Then the bandage is packed and sterilised. The bandage is intended to be applied directly to the wound.

### Example 6

1 g of chitosan-glucan (the dynamic viscosity is 30 Pa.s at the concentration of the solution of 2.5 %, at 25 °C and revolutions of 0.0314 rad/s, the glucosamine content is 0.15 (n/n)) and 0.5 g of sodium alginate having the molecular weight of 80 000 g/mol are dissolved in 100 ml of sterile inject water, PHMB in the form of a solution is added so that its final concentration in the solution with the chitosan-glucan and alginate is 0.1 % and the final solution is sterilised. The highly viscous gel is poured into a mould coated with a non-woven PP textile. The poured mixture is dried in a tray hot-air drier at 60°C for 24 hours. The foil thus prepared, immobilised on the non-woven PP textile, is intended to be applied directly to the wound, wherein it is applied to the wound with the side without the textile.

## Claims

1. A preparation for moist wound healing and prevention of a bandage adhesion to the wound, **characterised by** that it contains a pharmacologically suitable chitosan-glucan complex or a salt thereof, in a combination with one or more other polysaccharides or suitable salts thereof.

2. A preparation according to claim 1, **characterised by** that it further contains an antiseptic agent.

3. The preparation according to claim 1 or 2, **characterised by** that the pharmacologically suitable chitosan-glucan complex has the dynamic viscosity of a 2.5% solution at 25 °C and revolutions of 0.0314 rad/s comprised within the range from 0.1 to 100 Pa.s, the weight ratio of chitosan to glucan is within the range from 0.01:99.99 to 99.99:0.01; the ratio of the glucosamine units to the total number of monosaccharide units is within the range from 0 to 1, and is in its free form or bound in the form of a suitable salt.

4. The preparation according to any of claims 1 to 3, **characterised by** that the pharmacologically suitable salt is hydrochloride, lactate, acetate, propionate, succinate, glycolate or another water-soluble salt of an acid, or a mixture thereof.

5. The preparation according to any of claims 1 to 4, **characterised by** that the concentration of the pharmacologically suitable chitosan-glucan complex or a salt thereof is within the range from 0.01 to 100 % by weight of the dry matter, said other polysaccharide or polysaccharides are present in the concentration within the range from 0 to 99.99 % by weight of the dry matter and said antiseptic agent is present in the concentration of within the range from 0 to 50 % by weight of the dry matter.

6. The preparation according to claims 1 to 5, **characterised by** that the concentration of the pharmacologically suitable chitosan-glucan complex or a salt thereof is preferably within the range from 40 to 90 % by weight of the dry matter, said other polysaccharide or polysaccharides are preferably present in the concentration within the range from 10 to 60 % by weight of the dry matter and said antiseptic agent is present in the concentration of within the range from I to 25 % by weight of the dry matter.

7. The preparation according to claims 1 to 6, **characterised by** that said other polysaccharide or polysaccharides are selected from the group comprising free forms or pharmacologically suitable salts of hyaluronic acid, alginic acid, carboxymethyl cellulose, chitosan, oxidised cellulose, fucan, schizophyllan, carboxymethyl-, sulfoethyl- or another water-soluble 1,3-D-glucan or a mixture thereof.

8. The preparation according to claim 7, **characterised by** that said other polysaccharide is hyaluronic acid having the molecular weight within the range from 1 000 to 2 500 000 g/mol and being in its free form or in the form of a pharmacologically suitable salt which is a sodium, potassium, lithium, calcium, magnesium, zinc, cobalt, manganese or another salt or a mixture thereof.

9. The preparation according to claim 7, **characterised by** that said other polysaccharide is alginic acid having the molecular weight within the range from 1 000 to 1 000 000 g/mol and the ratio of the total number of D-mannuronic units to the total number of L-guluronic units within the range from 0.1 to 5, and being in its free form or in the form of a pharmacologically suitable salt which is a sodium, potassium, lithium, calcium, magnesium, zinc, cobalt, manganese or another salt or a mixture thereof.

10. The preparation according to claim 7, **characterised by** that said other polysaccharide is carboxymethyl cellulose having the molecular weight within the range from 1 000 to 1 500 000 g/mol and the substitution degree within the range from 0.1 to 3 expressed as the total number of carboxymethyl groups to the total number of monosaccharidic units, and being in its free form or in the form of a pharmacologically suitable salt which is a sodium, potassium, lithium, calcium, magnesium, zinc, cobalt, manganese or another salt or a mixture thereof.

11. The preparation according to claim 7, **characterised by** that said other polysaccharide is schizophyllan, i.e. β-1,3-D-glucan, having the molecular weight within the range from 1 000 to 2 000 000 g/mol.

12. The preparation according to claim 7, **characterised by** that said other polysaccharide is chitosan having the molecular weight within the range from 1 000 to 1 000 000 g/mol and the deacetylation degree within the range from 0.1 to 1 expressed as the total number of deacetylated glucosamine units to the total number of monosaccharidic units, and being in its free form or in the form of a pharmacologically suitable salt which is a hydrochloride, lactate, acetate, propionate, succinate, glycolate or another water-soluble salt of an acid, or a mixture thereof.

13. The preparation according to claim 7, **characterised by** that said other polysaccharide is the oxidised cellulose having the molecular weight within the range of 1 000 to 1 000 000 g/mol and the oxidation degree within the range of 0.05 to 1 expressed as the total number of glucuronic units to the total number of monosaccharidic units, and being in its free form or in the form of a pharmacologically suitable salt which is a sodium, potassium, lithium, calcium, magnesium, zinc, cobalt, manganese salt or another salt or a mixture thereof.

14. The preparation according to claim 7, **characterised by** that said other polysaccharide is fucan having the molecular weight within the range of 1 000 to 500 000 g/mol and the ratio of the fucose units to the total number of monosaccharide units at least 0.25 and the weight fraction of the sulfo groups at least 0.1 % by weight.

15. The preparation according to claim 7, **characterised by** that said other polysaccharide is carboxymethyl-β-1,3(1,6)-D-glucan having the molecular weight within the range of 1 000 to 1 500 000 g/mol and the substitution degree within the range of 0.05 to 3 expressed as the total amount of carboxymethyl groups to the total amount of monosaccharide units, and being in its free form or in the form of a pharmacologically suitable salt which is a sodium, potassium, lithium, calcium, magnesium, zinc, cobalt, manganese salt or another salt or a mixture thereof.

16. The preparation according to claim 7, **characterised by** that said other polysaccharide is sulfoethyl-β-1,3-D-glucan having the molecular weight within the range of 1 000 to 1 500 000 g/mol and the substitution degree within the range of 0.05 to 3 expressed as the total amount of sulfoethyl groups to the total amount of monosaccharide units, and being in its free form or in the form of a pharmacologically suitable salt which is a sodium, potassium, lithium, calcium, magnesium, zinc, cobalt, manganese salt or another salt or a mixture thereof.

17. The preparation according to claims 2, **characterised by** that said antiseptic agent is selected from the group comprising an antiseptic-acting glycol or mixtures thereof, a quarternary ammonium salt or mixtures thereof, a biguanidine derivative or mixtures thereof, octenidine or mixtures of its derivatives, iodine complexes such as iodine/potassium iodide or another complex with iodine, bismuttribromophenate, silver, silver sulfadiazine or another antiseptic-acting substances or mixtures thereof.

18. The preparation according to claims 1 to 5, **characterised by** that it is in the form of a foil, lyophilizate, a foil deposited on a suitable textile or a lyopbilizate deposited on a suitable textile or a combination of said forms.

19. The preparation according to claim 18, **characterised by** that said textile is made of the oxidised cellulose, polyamide PAD, polypropylene PP or another suitable polymer not exhibiting an adhesion to the wound, or a mixture thereof

## Patentansprüche

1. Zubereitung für Behandlung von feuchter Wunde und für Vorbeugung einer Adhäsion des Wundverbands an der Wunde, **dadurch gekennzeichnet, dass** die Zubereitung einen pharmakologisch annehmbaren Chitosan-Glukan-Komplex oder sein Salz kombiniert mit einem oder mehreren weiteren Polysacchariden oder ihren Salzen enthält.

2. Zubereitung nach Anspruch 1, **dadurch gekennzeichnet, dass** sie weiter ein antiseptisches Mittel enthält.

3. Zubereitung nach Anspruch 1 oder 2, **dadurch gekennzeichnet, dass** der pharmakologisch annehmbare Chitosan-Glukan-Komplex die dynamische Viskozität einer 2,5 % Lösung bei 25° und bei der Drehung 0,0314 rad/s im Bereich von 0,1 bis 100 Pa.s hat, das Massenverhältniss des Chitosans zu dem Glukan im Bereich von 0,01:99,99 bis 99,99:0,01 ist; das Verhältniss der Glucosamin-Einheiten zu der Gesamtzahl der Monosaccharid-Einheiten im Bereich von 0 bis 1 ist, und der Komplex in seiner freien Form oder in der Form eines annehmbaren Salzes ist.

4. Zubereitung nach einem der Ansprüche 1 bis 3, **dadurch gekennzeichnet, dass** das pharmakologisch annehmbare Salz Hydrochlorid, Lactat, Acetat, Propionat, Succinat, Glycolat oder ein anderes wasserlösliches Salz einer Säure, oder eine Mischung davon ist.

5. Zubereitung nach einem der Ansprüche 1 bis 4, **dadurch gekennzeichnet, dass** das die Konzentration des pharmakologisch annehmbaren Chitosan-Glukan-Komplexes oder seines Salzes im Bereich von 0,01 bis 100 % Gewichtsprozent der Trockensubstanz ist, das ein oder mehrere weitere Polysaccharide in einer Konzetration im Bereich von 0 bis 99.99 % Gewichtsprozent der Trockensubstanz anwesend sind, und das antiseptische Mittel in einer Konzetration im Bereich von 0 bis 50 % Gewichtsprozent der Trockensubstanz anwesend ist.

6. Zubereitung nach einem der Ansprüche 1 bis 5, **dadurch gekennzeichnet, dass** die Konzentration des pharmakologisch annehmbaren Chitosan-Glukan-Komplexes oder seines Salzes vorzugsweise im Bereich von 40 bis 90 % Gewichtsprozent der Trockensubstanz ist, das ein oder mehrere weitere Polysaccharide in einer Konzetration im Bereich von 10 bis 60 % Gewichtsprozent der Trockensubstanz anwesend sind, und das antiseptische Mittel in einer Konzetration im Bereich von 1 bis 25 % Gewichtsprozent der Trockensubstanz anwesend ist.

7. Zubereitung nach einem der Ansprüche 1 bis 6, **dadurch gekennzeichnet, dass** das ein oder mehrere weitere Polysaccharide aus einer freie Formen oder pharmakologisch annehmbaren Salze der Hyaluronsäure, Alginsäure, Carboxymethylcellulose, Chitosan, oxidierte Cellulose, Fucan, Schizophyllan, Carboxymethyl-, Sulfoethyl- oder ein anderes wasserlösliches 1,3-D-Glukan oder deren Mischungen umfassenden Gruppe gewählt ist.

8. Zubereitung nach dem Anspruch 7, **dadurch gekennzeichnet, dass** das weitere Polysaccharide eine Hyaluronsäure ist, die ihr Molekulargewicht im Bereich von 1 000 bis 2 500 000 g/mol hat und die in ihrer freien Form oder in der Form eines pharmakologisch annehmbaren Salzes, also Natrium-, Kalium-, Lithium-, Calcium-, Magnesium-, Zink-, Kobalt-, Mangan- oder eines anderen Salzes oder einer Mischung davon, ist.

9. Zubereitung nach dem Anspruch 7, **dadurch gekennzeichnet, dass** das weitere Polysaccharide eine Alginsäure ist, die ihr Molekulargewicht im Breich von 1 000 bis 1 000 000 g/mol und das Verhältnis der Gesamtzahl von D-Mannuronsäure-Einheiten zu der Gesamtzahl von L-Guluronsäure-Einheiten im Bereich von 0,1 bis 5 ist und die in ihrer freien Form oder in der Form eines pharmakologisch annehmbaren Salzes, also Natrium-, Kalium-, Lithium-, Calcium-, Magnesium-, Zink-, Kobalt-, Mangan- oder eines anderen Salzes oder einer Mischung davon, ist.

10. Zubereitung nach dem Anspruch 7, **dadurch gekennzeichnet, dass** das weitere Polysaccharide eine Carboxymethylcellulose ist, die ihr Molekulargewicht im Bereich von 1 000 bis 1 500 000 g/mol und den Substitutionsgrad im Bereich von 0,1 bis 3 ausgedrückt als die Gesamtzahl der Carboxymethylgruppen zu der Gesamtzahl der monosaccharidic Einheiten hat und die in ihrer freier Form oder in der Form eines pharmakologisch annehmbaren Salzes, also Natrium-, Kalium-, Lithium-, Calcium-, Magnesium-, Zink-, Kobalt-, Mangan- oder eines anderen Salzes oder einer Mischung davon, ist.

11. Zubereitung nach dem Anspruch 7, **dadurch gekennzeichnet, dass** das weitere Polysaccharide Schizophyllan, also β-1,3-D-Glukan, mit einer Molekulargewicht im Bereich von 1 000 bis 2 000 000 g/mol ist.

12. Zubereitung nach dem Anspruch 7, **dadurch gekennzeichnet, dass** das weitere Polysaccharide Chitosan mit einem Molekulargewicht im Bereich von 1000 bis 1 000 000 g/mol und mit einem Deacetylierungsgrad im Bereich von 0,1 bis 1 ausgedrückt als die Gesamtzahl von deacetilierten Glucosamin-Einheiten zu der Gesamtzahl von Monosaccharide-Einheiten, und in seiner freien Form oder in der Form eines pharmakologisch annehmbaren Salzes, also Hydrochlorid, Lactat, Acetat, Propionat, Succinat, Glycolat oder eines anderen wasserlöslichen Salzes einer Säure, oder einer Mischung davon ist.

13. Zubereitung nach dem Anspruch 7, **dadurch gekennzeichnet, dass** das weitere Polysaccharide eine oxidierte Cellulose ist, die ihr Molekulargewicht im Bereich von 1 000 bis 1 000 000 g/mol und den Oxidationsgrad im Bereich von 0,05 bis 1 ausgedrückt als die Gesamtzahl der Glucuron-Einheiten zu der Gesamtzahl der monosaccharidic Einheiten hat und die in ihrer freien Form oder in der Form eines pharmakologisch annehmbaren Salzes, also Natrium-, Kalium-, Lithium-, Calcium-, Magnesium-, Zink-, Kobalt-, Mangan- oder eines anderen Salzes oder einer Mischung davon, ist.

14. Zubereitung nach dem Anspruch 7, **dadurch gekennzeichnet, dass** das weitere Polysaccharide Fucan ist, das sein Molekulargewicht im Bereich von 1 000 bis 500 000 g/mol hat und die Verhältnisszahl der Gesamtzahl der Fucose-Einheiten zu der Gesamtzahl der Monosaccharide Einheiten mindenstens 0,25 ist und der Gewichtsanteil der Sulfogruppen mindestens 0,1 Gew. % ist.

15. Zubereitung nach dem Anspruch 7, **dadurch gekennzeichnet, dass** das weitere Polysaccharide Karboxymethyl-β-1,3(1,6)-D-Glukan ist, das sein Molekulargewicht im Bereich von 1 000 bis 1 500 000 g/mol hat und den Substitutionsgrad im Bereich von 0,05 bis 3 ausgedrückt als die Gesamtzahl der Karboxymethylgruppen zu der Gesamtzahl der monosaccharidic Einheiten hat und das in seiner freien Form oder in der Form eines pharmakologisch annehmbaren Salzes, also Natrium-, Kalium-, Lithium-, Calcium-, Magnesium-, Zink-, Kobalt-, Mangan- oder eines anderen Salzes oder einer Mischung davon, ist.

16. Zubereitung nach dem Anspruch 7, **dadurch gekennzeichnet, dass** das weitere Polysaccharide Karboxymethyl-β-1,3-D-Glukan ist, das sein Molekulargewicht im Bereich von 1 000 bis 1 500 000 g/mol hat und den Substitutionsgrad im Bereich von 0,05 bis 3 ausgedrückt als die Gesamtzahl der Sulfoethylgruppen zu der Gesamtzahl der monosaccharidic Einheiten hat und das in seiner freien Form oder in der Form eines pharmakologisch annehmbaren Salzes, also Natrium-, Kalium-, Lithium-, Calcium-, Magnesium-, Zink-, Kobalt-, Mangan- oder eines anderen Salzes oder einer Mischung davon, ist.

17. Zubereitung nach dem Anspruch 2, **dadurch gekennzeichnet, dass** das antiseptische Mittel aus einer Gruppe gewählt ist, die antiseptish wirkendes Glycol oder Mischungen davon, kvartäres Ammoniumsalz oder Mischungen davon, Biguanidinderivate oder Mischungen davon, Octenidin oder Mischungen von seinen Derivaten, Jodkomplexe, wie zum Beipsiel Jod-/Kaliumjodid oder ein weiteres Komplex mit Jod, Bismuttribromophenate, Silber, Silbersulfadiazine oder weitere antiseptisch wirkende Substanzen oder Mischungen davon beinhaltet.

18. Zubereitung nach den Ansprüchen 1 bis 5, **dadurch gekennzeichnet, dass** das sie in der Form von einer Folie, von einem Lyophilisaten, von einer auf einer passenden Textilie eingetragenen Folie oder von einem auf einer passenden Textilie eingetragenen Lyophilisaten oder in einer Kombination der genannten Formen ist.

19. Zubereitung nach dem Anspruch 18, **dadurch gekennzeichnet, dass** die Textilie aus einer oxidierten Zellulose, aus einem Polyamid PAD, Polypropylen PP oder aus einem weiteren passenden, keine Adhäsion an der Wunde aufweisenden Polymer oder aus Mischungen davon gemacht ist.

## Revendications

1. Une préparation pour la guérisson humide des plaies et pour la prévention de l'adhérence du bandage à la plaie, **caractérisé par** ce qu'elle contient un complex de chitosane-glucane pharmacologiquement acceptable ou un sel de celui-ci, en combinaison avec un ou plusieurs autres polysaccharides ou des sels appropriés de ceux-ci.

2. La préparation selon la revendication 1, **caractérisé par** ce qu'elle contient en outre un agent antiseptique.

3. La préparation selon la revendication 1 ou 2, **caractérisé par** ce que le complex de chitosane-glucane pharmacologiquement acceptable a la viscosité dynamique de la solution de 2.5% à 25 °C et aux révolutions 0.0314 rad/s entre 0.1 et 100 Pa.s, le ratio de masse de chitosane au glucane entre 0.01:99.99 et 99.99:0.01, le ratio des entités glucosamine au nombre total des entités polysaccharidiques entre 0 et 1, et il est en sa forme libre ou lié en forme d'un sel approprié.

4. La préparation selon quelconque des revendications 1 à 3, **caractérisé par** ce que le sel pharmacologiquement acceptable est hydrochloride, lactate, acetate, propionate, succinate, glycolate ou un autre sel hydrosoluble d'un acide, ou un mélange de ceux-ci.

5. La préparation selon quelconque des revendications 1 à 4, **caractérisé par** ce que la concentration du complex de chitosane-glucane pharmacologiquement acceptable ou du sel de celui-ci est entre 0.01 et 100 % de masse de la matière sèche, l'autre polysaccharide ou les autres polysaccharides sont présents dans la concentration entre 0 et 99.99 % de masse de la matière sèche et l'agent antiseptique est présent dans la concentration entre 0 et 50 % de masse de la matière sèche.

6. La préparation selon les revendications 1 à 5, **caractérisé par** ce que la concentration du complex de chitosane-glucane pharmacologiquement acceptable ou du sel de celui-ci est de préférence entre 40 et 90 % de masse de la matière sèche, l'autre polysaccharide ou les autres polysaccharides sont présents de préférence dans la concentration entre 10 et 60 % de masse de la matière sèche et l'agent antiseptique est présent dans la concentration entre 1 et 25 % de masse de la matière sèche.

7. La préparation selon les revendications 1 à 6, **caractérisé par** ce que l'autre polysaccharide ou les autres polysaccharides sont choisis du groupe comprenant les formes libres ou les sels pharmacologiquement acceptables de l'acide hyaluronique, l'acide alginique, carboxyméthyl cellulose, chitosane, la cellulose oxydée, fucane, schizophyllane, carboxyméthyl-, sulfoéthyl- ou un autre 1,3-D-glucane hydrosoluble ou un mélange de ceux-ci.

8. La préparation selon la revendication 7, **caractérisé par** ce que l'autre polysaccharide est l'acide hyaluronique ayant la masse moléculaire entre 1 000 et 2 500 000 g/mole et étant en sa forme libre ou en forme d'un sel pharmacologiquement acceptable qui est sodique, potassique, lithique, calcaire, magnésien, zincique, cobalteux, manganeux ou un autre sel ou un mélange de ceux-ci.

9. La préparation selon la revendication 7, **caractérisé par** ce que l'autre polysaccharide est l'acide alginique ayant la masse moléculaire entre 1 000 et 1 000 000 g/mole et le ratio du nombre total des entités D-mannuroniques au nombre total des entités L-glucuroniques entre 0.1 et 5, et étant en sa forme libre ou en forme d'un sel pharmacologiquement acceptable qui est sodique, potassique, lithique, calcaire, magnésien, zincique, cobalteux, manganeux ou un autre sel ou un mélange de ceux-ci.

10. La préparation selon la revendication 7, **caractérisé par** ce que l'autre polysaccharide est carboxyméthylcellulose ayant la masse moléculaire entre 1 000 et 1 500 000 g/mole et le degré de la substitution entre 0.1 et 3 exprimé en ratio du nombre total des groupes carboxyméthyls au nombre total des entités monosaccharidiques, et étant en sa forme libre ou en forme d'un sel pharmacologiquement acceptable qui est sodique, potassique, lithique, calcaire, magnésien, zincique, cobalteux, manganeux ou un autre sel ou un mélange de ceux-ci.

11. La préparation selon la revendication 7, **caractérisé par** ce que l'autre polysaccharide est schizophyllan, c.-à-d. β-1,3-D-glucane, ayant la masse moléculaire entre 1 000 et 2 000 000 g/mole.

12. La préparation selon la revendication 7, **caractérisé par** ce que l'autre polysaccharide est chitosane ayant la masse moléculaire entre 1 000 et 1 000 000 g/mole et le degré de la déacetylation entre 0.1 et 1 exprimé en ratio du nombre total des entités glucosamins déacetylés au nombre total des entités monosaccharidiques, et étant en sa forme libre ou en forme d'un sel pharmacologiquement acceptable qui est chlorhydrate, lactate, acétate, propionate, succinate, glycolate ou un autre sel hydrosoluble d'un acide ou un mélange de ceux-ci.

13. La préparation selon la revendication 7, **caractérisé par** ce que l'autre polysaccharide est cellulose oxydée ayant la masse moléculaire entre 1 000 et 1 000 000 g/mole et le degré de l'oxydation entre 0.05 et 1 exprimé en ratio du nombre total des entités glucuroniques au nombre total des entités monosaccharidiques, et étant en sa forme libre ou en forme d'un sel pharmacologiquement acceptable qui est sodique, potassique, lithique, calcaire, magnésien, zincique, cobalteux, manganeux ou un autre sel ou un mélange de ceux-ci.

14. La préparation selon la revendication 7, **caractérisé par** ce que l'autre polysaccharide est fucane ayant la masse moléculaire entre 1 000 et 500 000 g/mole et le ratio du nombre total des entités de fucose au nombre total des entités monosaccharidiques au moins 0.25 et la fraction de masse des groupes sulfo au moins 0.1 % de masse.

15. La préparation selon la revendication 7, **caractérisé par** ce que l'autre polysaccharide est carboxyméthyl-β-1,3(1,6)-D-glucane ayant la masse moléculaire entre 1 000 et 1 500 000 g/mole et le degré de la substitution entre 0.05 et 3 exprimé en ratio du nombre total des groupes carboxyméthyls au nombre total des entités monosaccharidiques, et étant en sa forme libre ou en forme d'un sel pharmacologiquement acceptable qui est sodique, potassique, lithique, calcaire, magnésien, zincique, cobalteux, manganeux ou un autre sel ou un mélange de ceux-ci.

16. La préparation selon la revendication 7, **caractérisé par** ce que l'autre polysaccharide est sulfoéthyl-β-1,3-D-glucane ayant la masse moléculaire entre 1 000 et 1 500 000 g/mole et le degré de la substitution entre 0.05 et 3 exprimé en ratio du nombre total des groupes sulfoéthyls au nombre total des entités monosaccharidiques, et étant en sa forme libre ou en forme d'un sel pharmacologiquement acceptable qui est sodique, potassique, lithique, calcaire, magnésien, zincique, cobalteux, manganeux ou un autre sel ou un mélange de ceux-ci.

17. La préparation selon la revendication 2, **caractérisé par** ce que l'agent antiseptique est choisi du groupe comprenant glycol qui fonctionne en manière antiseptique ou des mélanges de ce-ci, un sel quaternaire d'ammonium ou des mélanges de ce-ci, un dérivé du biguanidine ou des mélanges de ce-ci, un dérivé du octénidine ou des mélanges de ce-ci, des complexes de iode, par exemple iode/iodure de potassium ou un autre complexe avec iode, bismuttribromophénate, l'argent, sulfadiazine de l'argent ou les autres substances foncionnant en manière antiseptique ou des mélanges de ceux-ci.

18. La préparation selon quelquonque des revendications 1 à 5, **caractérisé par** ce que elle est en forme d'une feuille, un produit de la cryodessication, une feuille enduite sur un textile approprié ou une combinaison de cettes formes.

19. La préparation selon la revendication 18, **caractérisé par** ce que le textile est produit de la cellulose oxydée, polyamide PAD, polypropylene PP ou un autre polymer approprié qui ne présente pas de l'adhérence à la plaie, ou un mélange de ceux-ci.
